# EUROPEAN PATENT SPECIFICATION

(11) **EP 2 663 354 B1**
(45) Date of publication and mention of the grant of the patent: **17.08.2016**
(21) Application number: 11804772.9
(22) Date of filing: 22.12.2011
(51) Int. Cl.: A61M 16/20

(54) **DIAPHRAGM VALVE ASSEMBLY**
MEMBRANVENTILANORDNUNG
ENSEMBLE SOUPAPE À DIAPHRAGME

(30) Priority: 10.01.2011 GB 201100299
(43) Date of publication of application: 20.11.2013
(73) Proprietor: Intersurgical AG, Vaduz (LI)
(72) Inventor: MILLER, Andrew Neil, Berkshire RG45 6DU (GB)
(74) Representative: Adamson Jones
(86) International application number: PCT/GB2011/052556
(87) International publication number: WO 2012/095622

(56) References cited:
- EP-A2- 0 143 618
- GB-A- 1 551 226
- GB-A- 2 015 349
- US-A- 4 190 045
- US-A- 4 712 580
- US-A- 5 020 532
- US-A1- 2005 034 726
- US-A1- 2008 223 368

## Description

The present invention relates to a diaphragm valve and more particularly to a diaphragm valve assembly for use within a respiratory fluid supply system.

Machine controlled breathing apparatus such as ventilators rely on the use of valves within the fluid supply system or breathing circuit in order to regulate inhalation and expiration. Such equipment is widely used in medical applications to assist or control a patient's breathing cycle.

Diaphragm valves are used in such equipment. One such use of diaphragm valves is for expiration valves which serve to regulate fluid flow between a patient and a fluid supply apparatus such as a ventilator. Such valves are life-critical and so the valve function is of paramount importance.

The need for compliance with hygiene and infection control standards has resulted in a trend towards disposable devices in place of reusable counterparts, which must be sterilised between each use. However the use of disposable devices places increased emphasis on the unit cost of the device. This provides a dichotomy for engineers since the design of a device for the purpose of cost reduction can conflict with the critical functional requirements of the device.

An example of one such disposable device is described in UK Patent Application No. 0520343.5 (published as GB 2418973), which has a valve chamber and a control chamber separated by a diaphragm member. That device relies upon the provision of both a multi-part diaphragm member assembly to provide a first seal and also a separate sealing ring formation in the valve housing which cooperates with a lid in order to provide a second seal. The second seal serves to isolate the control chamber from external/ambient pressure.

Such an arrangement has a complex geometry and is subject to manufacturing tolerances between the multiple components which form the assembly. These factors add cost and complexity to a manufacturing and assembly process.

Despite being a disposable device, its design is also suitable for a re-usable device.

EP 0 143 618 discloses a diaphragm valve device in which the diaphragm comprises a flexible peripheral portion and a semi-rigid plunger. The plunger is disclosed as an insert that fits into and is retained by the flexible peripheral portion. The plunger has a projection adapted to move within a confined space to align the diaphragm and provide damping to its movement.

Further examples of diaphragm valve assemblies are disclosed in US 4 712 580, US 5 020 532, US 2005/034726 and US 4 190 045.

It is an aim of the present invention to provide a diaphragm valve assembly of reduced complexity whilst ensuring the functional requirements of the device are met.

According to a first aspect of the invention, there is provided a diaphragm member for a diaphragm valve assembly, said diaphragm member comprising a body portion formed of a first material and an outer portion formed of a second material, wherein the outer portion comprises a unitary mass of resilient material shaped to provide both a circumferential diaphragm flange for controlling actuation of the valve assembly in use in response to a pressure differential applied across diaphragm flange, and a peripheral sealing flange spaced from the diaphragm flange, wherein the body and outer portions are formed as a single member, and the peripheral sealing flange has an outer circumferential edge providing a circumferential sealing portion for selective cooperation with a valve seat in use in response to the actuation of the valve assembly.

According to a second aspect of the invention, there is provided a diaphragm valve assembly for a respiratory system comprising: a valve housing having a first port arranged for fluid communication with a fluid supply and an outlet spaced from the first port by a valve chamber; a diaphragm member according to the first aspect of the invention arranged for the selective control of flow between the first port and the outlet, wherein the valve housing has an opening across which the diaphragm member extends; and, a lid shaped to cooperate with the valve housing about said opening.

The lid may be shaped to cooperate with the valve housing so as to trap the diaphragm member there-between and thereby define a control chamber between the diaphragm member and the lid, said control chamber being isolated from the valve chamber by said diaphragm member, and wherein cooperation between the housing, the lid and the diaphragm there-between forms a first seal between the valve chamber and the control chamber and a second seal between the control chamber and the outside of the assembly.

In one embodiment the circumferential diaphragm flange of the diaphragm member comprises a peripheral edge and the diaphragm member is sandwiched between the valve housing and lid about said peripheral edge. The diaphragm member may be substantially circular in plan.

According to one particular embodiment, the fist seal is formed between the diaphragm member and the valve housing. The second seal may be formed between the diaphragm member and the lid. The first and second seals may be formed on respective first and second surfaces of the diaphragm member. The first and second seals may be formed on opposing sides of the diaphragm member. The first and second sides or surfaces of the diaphragm member may be separated by a peripheral edge.

The housing, diaphragm member and lid may comprise a common axis. Either or both of the first and second seals may be formed by linear compression of the diaphragm member between the housing and lid in a direction substantially parallel with said axis. The axis may comprise an alignment axis.

According to one embodiment, the lid comprises a control port for fluid communication with a pressure controller. The control port typically constitutes the only fluid communication port to the control chamber in use. The seal between the diaphragm member and lid typically seals the control chamber from the ports in the housing and the exterior of the assembly.

The lid may comprise a control port connector formation. The connector formation may be integrally formed with the lid. The connector formation and remainder of the lid may be formed of the same material. The connector formation may be co-formed with the lid for example by way of a moulding process.

Typically the connector formation is shaped so as to define a duct therein. The duct may pass from the control port through the connector formation to an open end for communication with the pressure controller. The connector formation may comprise a connector flange, which may be at the open end of the connector formation. The connector formation may comprise a neck portion extending between the lid surface and the flange.

The valve chamber may comprise a valve seat therein. The diaphragm member may be arranged for selective movement relative to the valve seat. The diaphragm member may be arranged for actuation between an open condition in which the diaphragm member is spaced from the valve seat such that the first port is in fluid communication with the outlet, and a closed condition in which contact between the diaphragm member and the valve seat interrupts flow between said first port and outlet

The housing may comprise a wall about said opening. The wall may comprise a sealing formation on an outwardly facing surface or edge thereof. The wall may be upstanding from an abutment formation or ridge which may be arranged about said opening.

The lid may comprise a peripheral wall arranged to surround the housing wall. The lid wall may have a diameter which is greater than that of the housing wall. The lid may form a push-fit, friction-fit, interference-fir or snap-fit engagement with the housing. The lid wall may overhang the housing wall and may have one or more engagement formations thereon which are shaped to oppose correspondingly shaped engagement formations on the housing wall. The lid wall may abut against the abutment formation on the housing which may serve to correctly align the lid and housing.

The lid may comprise a locating formation depending from an inside surface thereof. The locating formation may comprise a pin member which may be located substantially at or about the centre of the lid when viewed in plan. The locating formation may be arranged for location within a corresponding locating formation on the diaphragm member. The diaphragm member locating formation may comprise a recess. The recess typically has a closed end. The recess may terminate within the body of the diaphragm member.

The outer portion of the diaphragm member may surround the body portion. The outer portion is preferably formed of a resiliently deformable material, which may comprise a polymer, an elastomer or thermoplastic elastomer.

The diaphragm flange may comprise an actuation flange shaped for cooperation between the housing and lid to form the first and second seals. The diaphragm flange may support the diaphragm body portion and may be arranged to resiliently deform in response to a pressure differential between the valve and control chambers so as to reversibly actuate the diaphragm member between closed and open conditions.

The sealing flange may contact the valve seat when the diaphragm member is in the closed condition so as to form a seal between the first port and the outlet.

The diaphragm member may comprise a supporting flange spaced a short distance from the sealing flange. Upon actuation to the closed condition the sealing flange may resiliently deform under an applied pressure differential between the valve and control chambers, in a direction towards the supporting flange. In the closed condition, the sealing flange may contact the supporting flange such that the supporting flange inhibits or restricts further deformation of the sealing flange.

In accordance with the above embodiments, the diaphragm member may be entirely formed by a moulding process without the need for separate assembly of individual diaphragm components. The diaphragm flange and sealing flange may be uniformly or integrally formed of the same material.

The outer portion may be formed by a moulding process such as injection moulding. The body portion and outer portion may be formed by the same process. The body portion and outer portion may be co-formed by a moulding process. The body and outer portions may be formed by a so-called multi-shot injection moulding process. The body and outer portions may be formed by a two-shot injection moulding process.

The outer portion may comprise a sleeve portion from which the diaphragm flange and sealing flange depend. The sleeve portion may be arranged about the body portion. The sleeve portion may have an axis and the diaphragm and sealing flanges may be spaced along said axis.

The diaphragm flange may have a greater diameter than that of the sealing flange.

In one embodiment, the outer portion comprises a further flange which may comprise a supporting flange. The supporting flange may be adjacent the sealing flange. The supporting flange may be arranged between the diaphragm flange and the sealing flange. The supporting flange may be arranged a relatively short distance from the sealing flange compared to that of the diaphragm flange.

The supporting flange may be thicker than the sealing flange. The supporting flange may have a greater diameter than that of the sealing flange. The supporting flange may be stiffer than the sealing flange. The sealing flange may be arranged in use to deform upon contact with a corresponding valve seat and the supporting flange may be arranged in use to prevent or resist deformation of the sealing flange beyond a predetermined threshold. The sealing flange may be arranged to deform such that it comes into contact with the supporting flange upon application of a predetermined force thereto by a corresponding valve seat formation.

The supporting flange may be integrally or unitarily formed with either or both of the sealing flange and diaphragm flange.

According to a third aspect of the invention there is provided a method of manufacturinga respiratory diaphragm valve assembly, comprising: providing a valve housing having a first port arranged for fluid communication with a fluid supply, an outlet spaced from the first port by a valve chamber and an opening; arranging a diaphragm member to extend across the opening in the valve housing; and attaching a lid to the valve housing about said opening so as to trap the diaphragm member between the lid and valve housing, characterised by: co-forming a body portion and an outer portion of the diaphragm member as a single member by a moulding process, wherein the outer portion comprises a unitary mass of resilient material shaped to provide both a circumferential diaphragm flange for controlling actuation of the valve assembly in use in response to a pressure differential applied across diaphragm flange, and a peripheral sealing flange spaced from the diaphragm flange for selective cooperation with a valve seat in use in response to the actuation of the valve assembly, wherein the peripheral sealing flange has an outer circumferential edge providing a circumferential sealing portion.

The term 'gas' is used hereinafter to refer to the respiratory fluid passing through the diaphragm valve assembly. It will be appreciated by those skilled in the art that such a gas stream may comprise different gases, including vapour and may also comprise a proportion of liquid phase material. For example such a gas stream may include gases other than air and may be tailored to a desired humidity level.

Practicable embodiments of the invention are described by way of example below with reference to the accompanying drawings, of which:
Figure 1 shows a three-dimensional view of an assembled diaphragm valve according to an embodiment the present invention;
Figure 2 is an exploded three-dimensional view of the valve assembly of Figure 1;
Figure 3 shows a plan view of the valve assembly of figure 1; and,
Figure 4 shows a cross section through the plane A-A in figure 3.

The present invention relates to a diaphragm valve assembly and/or diaphragm member there-for which offers a simplified construction.

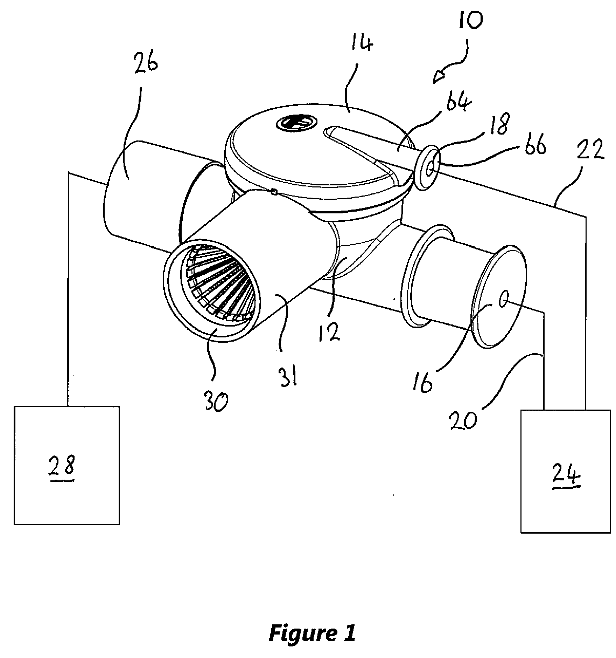

In Figure 1, there is shown an embodiment of the assembled diaphragm valve in use within a respiratory gas supply/control system. This type of valve may be referred to in the art as an expiration valve.

The diaphragm valve assembly 10 has a valve housing 12 and a lid 14, which define an internal valve geometry as will be described in further detail below. The lid may otherwise be described as a closure or cap. The valve assembly 10 has ports 16 and 18 arranged for fluid communication via respective gas paths 20 and 22 with mechanical ventilation equipment in the form of a ventilator 24. The ventilator 24 is of conventional design and has respective ports for connection with ports 16 and 18.

The details of the ventilator 24 itself will not be described here for conciseness since the operation of such equipment will be readily understood by a person skilled in the art.

The port 16 comprises a pressure monitoring port, which connects to pressure monitoring equipment in the ventilator 24.

The port 18 comprises a control port which connects to pressure control equipment in the ventilator.

A further port 26 is connected to, and in fluid communication with, a breathing system or circuit 28 for a user, who is typically a patient. Such a breathing system typically comprises one or more ducts which communicate gas to and from the user.

An exit port or outlet 30 opens to the outside of the valve assembly 10 and is thus exposed to ambient pressure in use.

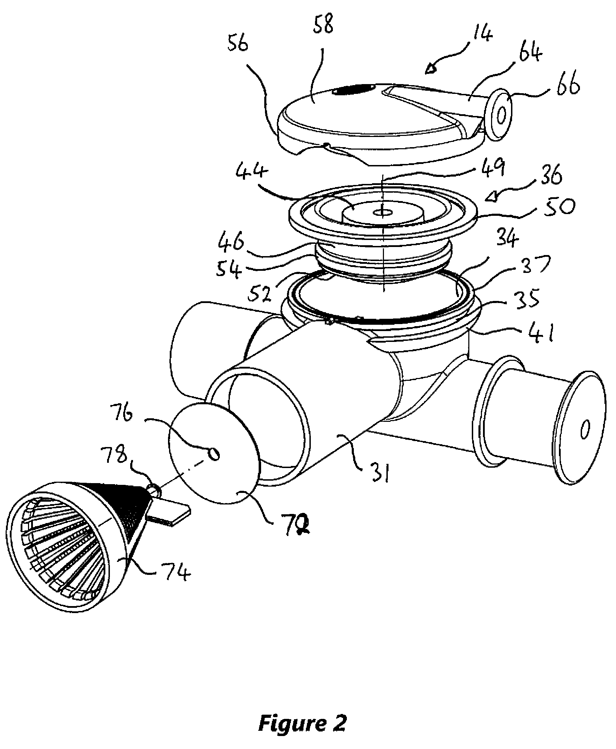

The structure of the device will now be described in further detail with reference to Figures 2 to 4.

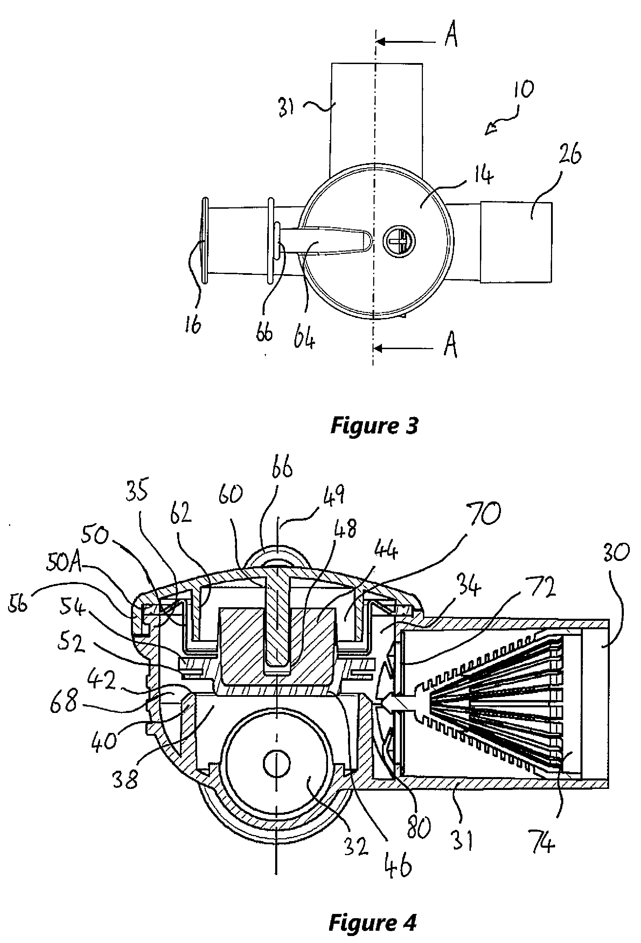

An internal duct 32 (shown in Figure 4) runs through the housing 12 from the pressure monitoring port 16 to the port 26 for connection to the breathing system 28. The internal duct passes through the housing 12, which is positioned between ports 16 and 26. In this embodiment, the duct 32 is generally linear.

The housing 12 is defined by a generally cylindrical outer surface or wall, which defines the inner space of the housing. The housing wall has openings therein for communication with ports 16, 26 and 30. The housing is shaped so as to define a generally circular opening 34 for cooperation with a diaphragm member 36 and the lid 14 as will be described below.

The housing 12 in the vicinity of the opening 34 has an upstanding wall portion 35 with an annular lip 37. A sealing formation in the form of an annular ridge or ring 39 protrudes upwardly from the outward-facing circumferential edge of the wall 35.

Part-way along the internal duct 32, inside the housing 12, there is provided an opening 38 in the internal duct about which is provided a substantially cylindrical upstanding wall formation in the form of a valve seat 40. The free edge or lip 42 of the valve seat is within the housing interior and may be profiled or otherwise shaped to cooperate in a sealing manner with the diaphragm valve member 36. The diaphragm member 36 is shaped for location in the housing 12 and comprises a central body portion 44 and an outer portion 46 disposed around the body portion. The outer portion is shaped to form a sleeve around the body portion, which is closed at one end.

Both the body 44 and outer portions 46 are formed of different materials as a single member or piece. This is achieved by a two stage or 'two-shot' injection moulding process, in which a mould has a first sprue for receiving a first material and a second separate sprue for receiving a second material. Thus the first and second material can be injected separately to form different parts of the moulded product. The properties of the first and second materials in liquid form, such as melting points, viscosities, etc) may differ sufficiently to allow consistent reproduction of a component, once solidified, having distinct predefined portions or stages consisting of said first and second materials. Also the process may allow a time delay between injection of the first and second materials such that a first portion of the component can at least partially solidify before injection of the second material.

The body portion is formed of a plastic/polymer material such as a suitable grade of polyethylene, polypropylene or polycarbonate. The outer portion is formed of a suitable flexible material such as an elastomer or silicone. The outer portion is preferably a thermoplastic elastomer, such as for example a SEBS based elastomer.

The body portion 44 is generally a solid cylinder save for a central recess 48 which is open at an exposed end of the body portion (i.e. an end that is not covered by the outer portion 46). The recess is aligned with an axis 49 of the diaphragm member 36 which is parallel with the direction of movement of the body portion 44 in use.

The outer portion 46 is shaped to define a sleeve from which depends a series of circumferential flanges 50, 52 and 54, all of which are formed as part of the substantially uniform mass of the outer portion by injection moulding as described above.

A first circumferential flange 50 of greatest diameter is formed so as to have a compliant annular mid-section and a thicker, more resilient rim or edge section 50A. The first flange 50 serves as the diaphragm element within the valve. That is to say, the first flange 50 is sufficiently compliant to be reversibly deformable upon application of operational pressure thereto so as to actuate the diaphragm member 36.

A second circumferential flange 52 provides a circumferential sealing portion and is shaped for cooperation with valve seat 40 in use. This sealing flange 52 is of smaller dimensions (i.e. diameter) than the diaphragm flange 50 and is spaced there-from along axis 49. The sealing flange is located towards the closed end of the outer portion 46.

A third circumferential flange 54 is spaced a short distance from sealing flange 52 and is located between the flanges 50 and 52. This intermediate flange 54 is positioned relative to the sealing flange 52 in order to aid in the sealing function of the diaphragm member 36 with the valve seat 40 as will be described below. The intermediate flange 54 is thicker in depth than the sealing flange 52 and therefore offers greater resilience to deformation upon application of a force thereto.

The lid 14 takes the form of a cap having a cover portion 58 and an outer circumferential rim or wall 56 which is upstanding from the cover. On the inside surface of the cover 58, there is provided a central pin formation 60, which is aligned with axis 49 when assembled, and annular wall 62 between the pin 60 and the outer rim 56. The rim formation 56 is recessed such that it fits over the duct 31 corresponding to outlet port 30.

Location formations are provided in the recessed portion of the rim and the outlet duct 31 in order to ensure correct alignment of the lid 14 on the housing 12.

The control port 18 is integrally formed with the lid 14. In this regard, the cover portion has an opening therein leading to a control duct formation 64 on the outer surface of the cover portion 58. The control duct 64 is elongate in form and radially aligned with respect to axis 49. The control duct protrudes radially outward of the circumferential rim 56 and terminates at its open end (i.e. at the control port) at a connector formation 66. The control duct 64 and connector formation 66 form a uniform piece with the lid 14.

The lid, including the structures 56, 58, 60, 62, 64 and 66, is formed by injection moulding and substantially comprises - or consists of - a plastic material.

The diaphragm valve assembly is assembled by placing the diaphragm member 36 on the housing 12 such that the diaphragm flange 50 covers the housing opening 34 and the rim 50A of the diaphragm flange 50 is seated on upstanding wall 35. In this configuration, the diaphragm member 36 and housing 12 share a common axis 49.

The lid 14 is then placed over the diaphragm member 36 onto the housing 12. The rim 56 of the lid is slightly larger in diameter than the wall 35 and annular lip 37 on the housing 12 such that the lid rim 56 cooperates with the housing lip 37 so as to form a push fit engagement therewith. In this manner the lid engages with the housing in a non-reversible manner such that the diaphragm valve assembly is a single-use or disposable device. In alternative embodiments, the lid and housing may have reversible connection formations, such as, for example threaded formations, in order to allow for disassembly and reuse of the valve.

The housing has an annular abutment flange 41 which protrudes outwardly from the wall 35, against which the lid rim 56 abuts when the lid and housing are correctly aligned for use.

The lid is placed on the housing such that each of the rim 56, the pin 60 and wall 62 extend inwardly into the internal cavity of the housing.

When placed on the housing, the lid presses on an upper edge of the diaphragm flange rim 50A, such that the rim 50A is sandwiched between the lid and housing. Thus the diaphragm flange is compressed between the lid and housing. In this manner there is defined a valve chamber 68 between the housing and inner side of diaphragm member 36, in which the valve seat 40 is located. There is also defined a control chamber 70 between the lid 14 and the outer side of diaphragm member 36.

The control 70 and valve 68 chambers are isolated from one another by the diaphragm member 36. This is achieved by a first annular seal formed between the lid and the outer side of diaphragm flange rim 50A and the separate annular seal formed between the housing and the inner side of diaphragm flange rim 50A. Thus separate seals are formed on opposing sides of the diaphragm flange 50. Ring-shaped formations may be formed on each of the lid and housing where contact is made with the diaphragm flange 50 in order to promote an effective seal. Such formations press into the resilient material of the flange rim 50 around its circumference.

The control duct 64 in the lid opens into the control chamber 70 in the assembled device.

The lid pin 60 extends into the recess 48 in the diaphragm member 36 to ensure correct alignment such that the lid and diaphragm member 36 share a common axis 49.

The annular wall 62 of the lid serves to align the lid and diaphragm flange 50 for use. The body portion 44 of diaphragm member 36 is smaller than the diameter of the annular wall 62 such that the body member 44 fits within the internal space defined by the wall.

Also in the assembly 10, a non-return valve 72 and filter member 74 are inserted into the exit duct 31. The non-return valve is formed of a simple disk which is cut from a silicone sheet material. An opening 76 is formed at the centre of the non-return valve for insertion of a locating formation 78 on the filter 78. As shown in Figure 4, the internal structure of the housing provides for locating formations 80 which abut the non-return valve 72 and/or filter tip 78 to ensure they are correctly seated in the housing.

In use, the pressure monitoring port 16 and control port 18 formation on the diaphragm valve assembly connect up to associated connection formation on a ventilator machine.

The diaphragm member 36 is shown in Figure 4 in a first, open, condition in which the sealing flange 52 is spaced from valve seat 40. In this condition, gas can flow from the internal duct 32 through the exit port 30 via the valve chamber 68. The gas exiting the device through port 30 may comprise air exhaled by the patient through port 26.

The diaphragm member 36 is actuated between open and closed conditions by a pressure differential between the control chamber 70 and duct 32 or valve chamber 68. In the open condition, as shown in Figure 4, the pressure in the duct 32 is greater than that in the control chamber 70. The pressure in the control port is controlled by pressure control equipment in the ventilator 24. In the event that the pressure in the control chamber 70 exceeds that in the duct 32, the diaphragm flange 50 will deform in response thereto and the diaphragm member 36 will be actuated to the closed condition.

In this manner the diaphragm member 36 can move back and forth in the direction of axis 49. The correct alignment of the diaphragm is maintained by the lid pin 60 in the recess 48 in the diaphragm body portion 44, which serves to constrain the motion of the diaphragm member 36. Accordingly the body portion 44 of the diaphragm member moves in a substantially linear, one-dimensional manner only. The annular wall 62 in the lid also limits the freedom of movement of the diaphragm flange 50.

The body portion is weighted, which serves to ensure correct operation of the valve. The portion is symmetrical about its axis such that the weight of the body acts in the direction of valve movement. The body portion supports the elastomeric material of the outer flanges. It also biases the diaphragm member in the at-rest condition.

When the diaphragm member is actuated to the closed condition, the sealing flange 52 first comes into contact with the open edge 42 of the valve seat 40. The material properties and dimensions of the sealing flange allow the sealing flange to deform under the pressure applied to control chamber 70 such that the sealing flange is pushed towards the supporting flange 54. The supporting flange then resists further deformation of the sealing flange 52 under the applied pressure. The trapping of the sealing flange in this manner between the valve seat 40 and the supporting flange 54 provides a particularly effective seal. This action is then reversed when the pressure in the duct 32 rises sufficiently to actuate the diaphragm member into the open condition as shown in Figure 4.

Gas is substantially prevented from re-entering the housing from outside by the non-return valve 72. This also provides a flow restriction or slight back pressure when gas is exiting the valve assembly 10 via port 30 to maintain the diaphragm member in the open condition.

The pressure in the duct 32 is monitored by control equipment in the ventilator via port 16 and, using suitable processing means, the ventilator can control the pressure applied to control port 70 in order to ensure the desired breathing cycle is established or maintained.

The arrangement described above provides for suitable operation of a diaphragm valve in a manner which facilitates simple, cost-effective assembly.

Whilst the assembled diaphragm valve sown in Figures 1 to 4 is shown in a particular orientation, in use it would in fact be oriented with the lid on the underside. The valve assembly could operate in any orientation provided suitable connections can be made to the ventilator and patient breathing system. Any references made above to relative spatial terms, such as 'upper', 'lower', 'above', 'below', 'upstanding', or the like, should be construed accordingly.

## Claims

1. A diaphragm member (36) for a respiratory diaphragm valve assembly (10), said diaphragm member (36) comprising:
a body portion (44) formed of a first material and
an outer portion (46) formed of a second material, wherein the outer portion (46) comprises a unitary mass of resilient material shaped to provide both
a circumferential diaphragm flange (50) for controlling actuation of the valve assembly (10) in use in response to a pressure differential applied across diaphragm flange (50), and
a peripheral sealing flange (52) spaced from the diaphragm flange (50),
**characterised in that**
the body (44) and outer (46) portions are formed as a single member, and
the peripheral sealing flange (52) has an outer circumferential edge providing a circumferential sealing portion for selective cooperation with a valve seat (40) in use in response to the actuation of the valve assembly (10).

2. A diaphragm member (36) according to claim 1 further comprising a supporting flange (54) adjacent the sealing flange (52) and arranged to resist deformation of the sealing flange (52) in use beyond a predetermined limit.

3. A diaphragm member (36) according to claim 2 wherein the outer portion (46) is shaped to provide the supporting flange (54) which is thicker than the sealing flange (52).

4. A diaphragm member (36) according to any preceding claim, wherein the outer portion (46) comprises a uniform mass of elastomeric material.

5. A diaphragm member (36) according to any preceding claim, wherein the body portion (44) is harder than the outer portion (46).

6. A diaphragm member (36) according to any preceding claim, wherein the diaphragm member (36) has an axis (49) and the body portion (44) has a recess (48) aligned with said axis (49).

7. A diaphragm member (36) according to any preceding claim, wherein the diaphragm flange (50) and the sealing flange (52) are arranged to be resiliently deformable in use.

8. A diaphragm member (36) according to any preceding claim, wherein the diaphragm member (36) is injection moulded and the body portion (44) and outer portion (46) comprise different stages thereof.

9. A diaphragm member (36) according to any preceding claim, wherein the outer portion (46) comprises a sleeve portion arranged about the body portion (44) and from which the diaphragm flange (50) and sealing flange (52) depend.

10. A diaphragm member (36) according to any preceding claim, wherein the diaphragm flange (50) has an outer circumferential edge and the diaphragm flange (50) has a greater diameter than the sealing flange (52).

11. A diaphragm member (36) according to any preceding claim, wherein the diaphragm flange (50) has an intermediate portion and an outer rim portion (50A), the rim portion (50A) being thicker in depth than the intermediate portion.

12. A diaphragm valve assembly (10) for a respiratory system comprising:
a valve housing (12) having a first port (26) arranged for fluid communication with a fluid supply and an outlet (30) spaced from the first port (26) by a valve chamber (68);
a diaphragm member (36) according to any preceding claim arranged for the selective control of flow between the first port (26) and the outlet (30), wherein the valve housing (12) has an opening (34) across which the diaphragm member (36) extends; and,
a lid (14) shaped to cooperate with the valve housing (12) about said opening (34).

13. A diaphragm valve assembly (10) according to claim 12, wherein lid (14) is shaped to cooperate with the valve housing (12) about said opening (34) so as to trap the diaphragm member (36) there-between and thereby define a control chamber (70) between the diaphragm member (36) and the lid (14), said control chamber (70) being isolated from the valve chamber (68) by said diaphragm member (36), and wherein cooperation between the lid (14) and diaphragm member (36) forms a seal between the control chamber (70) and the outside of the assembly (10).

14. A diaphragm valve assembly (10) according to claim 13, wherein the lid (14) comprises an alignment pin (6) located in a correspondingly shaped recess (48) in the diaphragm member (36).

15. A method of manufacturing a respiratory diaphragm valve assembly (10), comprising:
providing a valve housing (12) having a first port (26) arranged for fluid communication with a fluid supply, an outlet (30) spaced from the first port (26) by a valve chamber (68) and an opening (34);
arranging a diaphragm member (36) to extend across the opening (34) in the valve housing (12); and
attaching a lid (14) to the valve housing (12) about said opening (34) so as to trap the diaphragm member (36) between the lid (12) and valve housing (12)
**characterised by**
co-forming a body portion (44) and an outer portion (46) of the diaphragm member (36) as a single member by a moulding process, wherein the outer portion (46) comprises a unitary mass of resilient material shaped to provide both a circumferential diaphragm flange (50) for controlling actuation of the valve assembly (10) in use in response to a pressure differential applied across diaphragm flange (50), and a peripheral sealing flange (52) spaced from the diaphragm flange (50) for selective cooperation with a valve seat (40) in use in response to the actuation of the valve assembly (10), wherein the peripheral sealing flange (52) has an outer circumferential edge providing a circumferential sealing portion.

## Patentansprüche

1. Membranglied (36) für eine respiratorische Membranventilanordnung (10), wobei das Membranglied (36) umfasst:
einen aus einem ersten Material gebildeten Körperteil (44) und
einen aus einem zweiten Material gebildeten Außenteil (46), wobei der Außenteil (46) eine einheitliche Masse aus elastischem Material umfasst, die so geformt ist, dass sie im Gebrauch sowohl einen umlaufenden Membranflansch (50) zum Steuern der Betätigung der Ventilanordnung (10) als Reaktion auf ein Druckdifferential, das auf den Membranflansch (50) angelegt wird, als auch
einen peripheren Abdichtungsflansch (52), der von dem Membranflansch (50) beabstandet ist, bereitstellt,
**dadurch gekennzeichnet, dass**
der Körper- (44) und der Außenteil (46) als ein einzelnes Glied gebildet sind, und
der periphere Abdichtungsflansch (52) eine äußere umlaufende Kante hat, die im Gebrauch einen umlaufenden Abdichtungsteil zur selektiven Kooperation mit einem Ventilsitz (40) als Reaktion auf die Betätigung der Ventilzusammensetzung (10) bereitstellt.

2. Membranglied (36) nach Anspruch 1, weiter umfassend einen Stützflansch (54), der an den Abdichtungsflansch (52) angrenzt und so angeordnet ist, dass er im Gebrauch Verformung des Abdichtungsflansches (52) über einer vorher festgelegten Grenze widersteht.

3. Membranglied (36) nach Anspruch 2, wobei der Außenteil (46) so geformt ist, dass er den Stützflansch bereitstellt (54), der dicker ist als der Abdichtungsflansch (52).

4. Membranglied (36) nach einem der vorhergehenden Ansprüche, wobei der Außenteil (46) eine einheitliche Masse aus elastomerem Material umfasst.

5. Membranglied (36) nach einem der vorhergehenden Ansprüche, wobei der Körperteil (44) härter ist als der Außenteil (46).

6. Membranglied (36) nach einem der vorhergehenden Ansprüche, wobei das Membranglied (36) eine Achse (49) aufweist und der Körperteil (44) eine an der Achse (49) ausgerichtete Ausbuchtung (48) aufweist.

7. Membranglied (36) nach einem der vorhergehenden Ansprüche, wobei der Membranflansch (50) und der Abdichtungsflansch (52) so angeordnet sind, dass sie im Gebrauch elastisch verformbar sind.

8. Membranglied (36) nach einem der vorhergehenden Ansprüche, wobei das Membranglied (36) spritzgegossen ist und der Körperteil (44) und der Außenteil (46) verschiedene Stufen davon umfassen.

9. Membranglied (36) nach einem der vorhergehenden Ansprüche, wobei der Außenteil (46) einen Hülsenteil umfasst, der um den Körperteil (44) angeordnet ist und von dem der Membranflansch (50) und der Abdichtungsflansch (52) abhängen.

10. Membranglied (36) nach einem der vorhergehenden Ansprüche, wobei der Membranflansch (50) eine äußere umlaufende Kante aufweist und der Membranflansch (50) einen größeren Durchmesser aufweist als der Abdichtungsflansch (52).

11. Membranglied (36) nach einem der vorhergehenden Ansprüche, wobei der Membranflansch (50) einen Zwischenteil und einen Außenrandteil (50A) aufweist, wobei der Randteil (50A) in der Tiefe dicker ist als der Zwischenteil.

12. Membranventilanordnung (10) für ein respiratorisches System, umfassend:
ein Ventilgehäuse (12) mit einem ersten, zur fluidischen Kommunikation mit einer Fluidversorgung angeordneten Anschluss (26) und einem von dem ersten Anschluss (26) durch eine Ventilkammer (68) beabstandeten Auslass (30);
ein Membranglied (36) nach einem der vorhergehenden Ansprüche, das zur selektiven Steuerung von Strömung zwischen dem ersten Anschluss (26) und dem Auslass (30) angeordnet ist, wobei das Ventilgehäuse (12) eine Öffnung (34) aufweist, über die sich das Membranglied (36) erstreckt; und
eine Abdeckung (14), die geformt ist, um mit dem Ventilgehäuse (12) um die Öffnung (34) zu kooperieren.

13. Membranventilanordnung (10) nach Anspruch 12, wobei die Abdeckung (14) geformt ist, um mit dem Ventilgehäuse (12) um die Öffnung (34) zu kooperieren, um das Membranglied (36) dazwischen einzuschließen und dadurch eine Steuerungskammer (70) zwischen dem Membranglied (36) und der Abdeckung (14) zu definieren, wobei die Steuerungskammer (70) durch das Membranglied (36) von der Ventilkammer (68) isoliert ist, und wobei Kooperation zwischen der Abdeckung (14) und dem Membranglied (36) eine Abdichtung zwischen der Steuerungskammer (70) und dem Äußeren der Anordnung (10) bildet.

14. Membranventilanordnung (10) nach Anspruch 13, wobei die Abdeckung (14) einen Ausrichtungsstift (6) umfasst, der sich in einer entsprechend geformten Aussparung (48) in dem Membranglied (36) befindet.

15. Verfahren zum Herstellen einer respiratorischen Membranventilanordnung (10), umfassend:
Bereitstellen eines Ventilgehäuses (12) mit einem ersten Anschluss (26), der zur fluidischen Kommunikation mit einer Fluidversorgung angeordnet ist, einem Auslass (30), der von dem ersten Anschluss (26) durch eine Ventilkammer (68) beabstandet ist, und einer Öffnung (34);
Anordnen eines Membranglieds (36), so dass es sich über die Öffnung (34) in dem Ventilgehäuse (12) erstreckt; und
Befestigen einer Abdeckung (14) an dem Ventilgehäuse (12) um die Öffnung (34), um das Ventilglied (36) zwischen der Abdeckung (14) und dem Ventilgehäuse (12) einzuschließen,
**gekennzeichnet durch**
gemeinsames Bilden eines Körperteils (44) und eines Außenteils (46) des Membranglieds (36) als ein einzelnes Glied **durch** einen Gießprozess, wobei der Außenteil (46) eine einheitliche Masse aus elastischem Material umfasst, die so geformt ist, dass sie sowohl einen umlaufenden Membranflansch (50), um im Gebrauch die Betätigung der Ventilanordnung (10) als Reaktion auf ein Druckdifferential, das auf den Membranflansch (50) angelegt wird, zu steuern, als auch einen peripheren Abdichtungsflansch (52), der von dem Membranflansch (50) beabstandet ist, um im Gebrauch als Reaktion auf die Betätigung der Ventilanordnung (10) selektiv mit einem Ventilsitz (40) zu kooperieren, bereitstellt, wobei der periphere Abdichtungsflansch (52) eine äußere umlaufende Kante aufweist, die einen umlaufenden Abdichtungsteil bereitstellt.

## Revendications

1. Élément diaphragme (36) destiné à un ensemble soupape à diaphragme respiratoire (10), ledit élément diaphragme (36) comprenant :
une partie corps (44) formée d'un premier matériau et
une partie extérieure (46) formée d'un second matériau, dans lequel la partie extérieure (46) comprend une masse unitaire de matériau élastique formée pour fournir à la fois un rebord circonférentiel (50) de diaphragme permettant, en fonctionnement, de commander la mise en oeuvre dudit ensemble soupape (10) en réponse à un différentiel de pression appliqué de part et d'autre du rebord (50) de diaphragme, et
un rebord d'étanchéité périphérique (52) espacé du rebord (50) de diaphragme,
**caractérisé en ce que**
les parties corps (44) et extérieure (46) sont formées comme un élément unique, et
le rebord d'étanchéité périphérique (52) comporte un bord circonférentiel extérieur fournissant, en utilisation, une partie d'étanchéité circonférentielle de coopération sélective avec un siège (40) de soupape en réponse à la mise en oeuvre de l'ensemble soupape (10).

2. Élément diaphragme (36) selon la revendication 1, comprenant en outre un rebord de support (54) adjacent au rebord d'étanchéité (52) et conçu pour, en utilisation, résister à une déformation du rebord d'étanchéité (52) au-delà d'une limite prédéfinie.

3. Élément diaphragme (36) selon la revendication 2, dans lequel la partie extérieure (46) est formée pour fournir le rebord de support (54) qui est plus épais que le rebord d'étanchéité (52).

4. Élément diaphragme (36) selon l'une quelconque des revendications précédentes, dans lequel la partie extérieure (46) comprend une masse uniforme de matériau élastomère.

5. Élément diaphragme (36) selon l'une quelconque des revendications précédentes, dans lequel la partie corps (44) est plus dure que la partie extérieure (46).

6. Élément diaphragme (36) selon l'une quelconque des revendications précédentes, dans lequel l'élément diaphragme (36) a un axe (49) et la partie corps (44) comporte un évidement (48) aligné avec ledit axe (49).

7. Élément diaphragme (36) selon l'une quelconque des revendications précédentes, dans lequel le rebord (50) de diaphragme et le rebord d'étanchéité (52) sont conçus pour pouvoir se déformer élastiquement lors de l'utilisation.

8. Élément diaphragme (36) selon l'une quelconque des revendications précédentes, dans lequel l'élément diaphragme (36) est moulé par injection et la partie corps (44) et la partie extérieure (46) comprennent des étages différents de ce dernier.

9. Élément diaphragme (36) selon l'une quelconque des revendications précédentes, dans lequel la partie extérieure (46) comprend une partie manchon disposée autour de la partie corps (44) et de laquelle dépendent le rebord (50) de diaphragme et le rebord d'étanchéité (52).

10. Élément diaphragme (36) selon l'une quelconque des revendications précédentes, dans lequel le rebord (50) de diaphragme comporte un bord circonférentiel extérieur et le rebord (50) de diaphragme a un diamètre plus grand que le rebord d'étanchéité (52).

11. Élément diaphragme (36) selon l'une quelconque des revendications précédentes, dans lequel le rebord (50) de diaphragme comporte une partie intermédiaire et une partie bord extérieure (50A), la partie bord (50A) étant plus épaisse que la partie intermédiaire dans la direction de la profondeur.

12. Ensemble soupape à diaphragme (10) de système respiratoire, comprenant :
un boîtier (12) de soupape comportant un premier orifice (26) conçu pour une communication fluidique avec une alimentation en fluide et une sortie (30) espacée du premier orifice (26) par une chambre (68) de soupape ;
un élément diaphragme (36) selon l'une quelconque des revendications précédentes, conçu pour réguler sélectivement le flux entre le premier orifice (26) et la sortie (30), dans lequel le boîtier (12) de soupape comporte une ouverture (34) de part et d'autre de laquelle s'étend l'élément diaphragme (36) ; et
un couvercle (14) formé pour coopérer avec le boîtier (12) de soupape autour de ladite ouverture (34).

13. Ensemble soupape à diaphragme (10) selon la revendication 12, dans lequel le couvercle (14) est formé pour coopérer avec le boîtier (12) de soupape autour de ladite ouverture (34) de façon à y piéger l'élément diaphragme (36) et ainsi définir une chambre de régulation (70) entre l'élément diaphragme (36) et le couvercle (14), ladite chambre de régulation (70) étant isolée de la chambre (68) de soupape par ledit élément diaphragme (36), et dans lequel la coopération entre le couvercle (14) et l'élément diaphragme (36) forme un joint d'étanchéité entre la chambre de régulation (70) et l'extérieur de l'ensemble (10).

14. Ensemble soupape à diaphragme (10) selon la revendication 13, dans lequel le couvercle (14) comprend une broche d'alignement (6) logée dans un évidement formé de manière correspondante (48) dans l'élément diaphragme (36).

15. Procédé de fabrication d'un ensemble soupape à diaphragme (10), consistant à :
concevoir un boîtier (12) de soupape comportant un premier orifice (26) conçu pour une communication fluidique avec une alimentation en fluide, une sortie (30) espacée du premier orifice (26) par une chambre (68) de soupape, et une ouverture (34) ;
disposer un élément diaphragme (36) pour qu'il s'étende de part et d'autre de l'ouverture (34) dans le boîtier (12) de soupape ; et
assujettir un couvercle (14) au boîtier (12) de soupape autour de ladite ouverture (34) de façon à piéger l'élément diaphragme (36) entre le couvercle (14) et le boîtier (12) de soupape
**caractérisé par** :
la formation conjointe d'une partie corps (44) et d'une partie extérieure (46) de l'élément diaphragme (36) comme un élément unique par un traitement de moulage, dans lequel la partie extérieure (46) comprend une masse unitaire de matériau élastique formée pour fournir à la fois un rebord circonférentiel (50) de diaphragme permettant, en utilisation, de commander la mise en oeuvre de l'ensemble soupape (10) en réponse à un différentiel de pression appliqué de part et d'autre d'un rebord (50) de diaphragme, et un rebord d'étanchéité périphérique (52) espacé du rebord (50) de diaphragme pour, en utilisation, établir une coopération sélective avec un siège (40) de soupape en réponse à la mise en oeuvre de l'ensemble soupape (10), dans lequel le rebord d'étanchéité périphérique (52) comporte un bord circonférentiel extérieur fournissant une partie d'étanchéité circonférentielle.
